# EUROPÄISCHE PATENTSCHRIFT

(11) **EP 0 681 565 B1**
(45) Veröffentlichungstag und Bekanntmachung des Hinweises auf die Patenterteilung: **18.06.1997**
(21) Anmeldenummer: 94905102.3
(22) Anmeldetag: 21.01.1994
(51) Int. Cl.: C07C 53/128, C07C 51/41, C07C 51/295

(54) **VERFAHREN ZUM HERSTELLEN ALPHA-VERZWEIGTER ALIPHATISCHER MONOCARBONSÄUREN**
PROCESS FOR PREPARING ALPHA-BRANCHED ALIPHATIC MONOCARBOXYLIC ACIDS
PROCEDE DE FABRICATION D'ACIDES MONOCARBOXYLIQUES ALIPHATIQUES ALPHA-RAMIFIES

(30) Priorität: 29.01.1993 DE 4302463
(43) Veröffentlichungstag der Anmeldung: 15.11.1995
(73) Patentinhaber: Henkel Kommanditgesellschaft auf Aktien, 40191 Düsseldorf (DE)
(72) Erfinder: MÜLLER, Gerhard, D-40239 Düsseldorf (DE); GUTSCHE, Bernhard, D-40721 Hilden (DE); SCHMID, Karl-Heinz, D-40822 Mettmann (DE); BONGARDT, Frank, D-40591 Düsseldorf (DE); JEROMIN, Lutz, D-40723 Hilden (DE); PEUKERT, Eberhard, D-40724 Hilden (DE); FRANKENBACH, Hermann, D-91180 Heideck (DE)
(86) Internationale Anmeldenummer: EP9400155
(87) Internationale Veröffentlichungsnummer: WO9417026

(56) Entgegenhaltungen:
- EP-A- 0 031 694
- DE-A- 2 357 865
- GB-A- 1 174 975
- US-A- 2 293 649

## Beschreibung

Die Erfindung betrifft ein Verfahren zum Herstellen α-verzweigter aliphatischer Monocarbonsäuren mit 12 bis 48 Kohlenstoffatomen. Dabei überführt man in einer ersten Verfahrensstufe a α-verzweigte aliphatische einwertige Alkohole (Guerbetalkohole) in Gegenwart von Alkalihydroxid in die Alkalisalze der entsprechenden α-verzweigten aliphatischen Monocarbonsäuren und setzt die α-verzweigten aliphatischen Monocarbonsäuren in einer zweiten Verfahrensstufe b aus den Alkalisalzen durch Seifenspaltung frei.

In der fettchemischen Industrie werden mit der Guerbet-Reaktion aus unverzweigten, gesättigten primären Alkoholen der Form R-CH₂-CH₂OH α-verzweigte Alkohole der Form 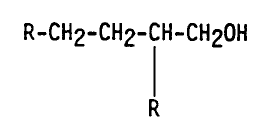 hergestellt, wobei R einen Kohlenwasserstoffrest mit 4 bis 20 Kohlenstoffatomen darstellt.

Diese Alkohole und ihre Derivate werden in einer Vielzahl von Rezepturen der Schmierstoffindustrie, der Kosmetik sowie der Textilpflege eingesetzt.

Bei den Fettsäuren sind ähnlich verzweigte Produkte bisher großtechnisch nicht herstellbar gewesen, obwohl für diese Fettsäuren und ihre Derivate entsprechende Anwendungsmöglichkeiten gegeben sind. Besonders die Ester mit Mehrfach-Alkoholen wie Pentaerythrit oder Trimethylolpropan zeichnen sich vorteilhaft durch niedrige Dampfdrücke und niedrige Stockpunkte aus.

Es hat daher nicht an Versuchen gefehlt, Herstellverfahren für die verzweigten Fettsäuren zu finden.

In einem in der DE-A-2 320 461 beschriebenen Verfahren der eingangs genannten Art wird zuerst eine Umsetzung von Guerbetalkoholen mit Alkali zu den carbonsauren Salzen vorgeschlagen. Die Darstellung der entsprechenden Monocarbonsäure gelingt anschließend über eine Seifenspaltung mit Mineralsäuren. Die erste Stufe dieses Verfahrens wird in einer oxidativen Alkalischmelze bei entsprechend hohen Temperaturen durchgeführt. Aufgrund der Schmelzpunkte von NaOH bzw. KOH, die aus wirtschaflichen Gründen eingesetzt werden, sind daher Betriebstemperaturen von mehr als 370°C erforderlich.

Auch die Stockpunkte der Seifen, die nach diesem Stand der Technik in flüssiger Form vorliegen müssen, um aus dem Reaktionsgemisch abgetrennt zu werden, liegen oberhalb von 340 °C. Der bei der Reaktion entstehende Wasserstoff führt bei der erhöhten Temperatur zu Sicherheitsproblemen. Neben der Bildung von Verblockungen in den Rohrleitungen stellte die hohe Korrosivität der Alkalischmelze gegenüber nahezu allen bekannten Werkstoffen ein Haupthindernis auf dem Weg zur großtechnischen Durchführung dieses Verfahrens dar.

In einem aus EP 31 694 B1 bekannten Verfahren zur Herstellung von Salzen von Carbonsäuren aus den entsprechenden Alkoholen und einer Base wird die Entstehung von festen Reaktionsprodukten und das Aufschäumen der Reaktionsmischung bei der alkalischen Oxidation der Alkohole durch Einsatz von inerten Viskositätserniedrigern verhindert. Vorgegeben wird hier vor Beginn der Reaktion eine Mischung der Base und eines Katalysator in einer inerten Verdünnungsflüssigkeit. Erst danach wird der umzusetzende Alkohol dieser Mischung unter Reaktionsbedingungen zudosiert. Die Zugabe des Verdünnungsmittels schon vor der Reaktion führt aber zur Absenkung der Raumzeitausbeute auf Werte zwischen 20 bis 80 %.

Die in den Vergleichsbeispielen in EP 31 694 B1 genannte Wasserzugabe dient ausschließlich dazu, die Verseifungsreaktion und damit die Wasserstoffbildung zu verlangsamen und so ein Aufschäumen zu verhindern.
Das in der EP 31 694 B1 genannte Kriterium zur Auswahl des Verdünnungsmittels ist die bei Reaktionsbedingungen nur geringe Flüchtigkeit, so daß das Verdünnungsmittel in der flüssigen Phase verbleibt.

Der Erfindung liegt die Aufgabe zugrunde, ein großtechnisch durchführbares Verfahren zu entwickeln, bei dem das genannte Problem des Aufschäumens gelöst ist, ohne daß der Nachteil einer Verringerung der Raumzeitausbeute in Kauf genommen werden muß. Dazu schlägt die Erfindung vor, daß man die Verfahrensstufe a, nämlich die Überführung der Guerbetalkohole in die Alkalisalze der entsprechenden Monocarbonsäuren, als Reaktion zwischen festem Alkalihydroxid und flüssigem Alkohol durchführt und dem Reaktionsgemisch nach Abschluß dieser Reaktion ein inertes Verdünnungsmittel zum Absenken der Viskosität zugibt. Insbesondere führt man das Verfahren kontinuierlich durch.

Die Wahl des Viskositätserniedrigers und Verdünnungsmittels richtet sich bevorzugt nach der an die Herstellung der Salze der verzweigten Monocarbonsäuren anschließenden Derivatisierung.

Im erfindungsgemäßen Verfahren werden die Einsatzstoffe dem Reaktor im kalten Zustand gemeinsam und vollständig zugeführt. Der Alkali, vorzugsweise NaOH in fester Form, läßt sich gegenüber dem oben genannten Schmelze-Verfahren in einfacher Weise genau dosieren.

Bereits ab einer Reaktortemperatur von 200 bis 250 °C beginnt während des Aufheizens die Reaktion zwischen flüssigem Alkohol und dem festen Alkalihydroxid. Bei dieser Reaktion wird Wasserstoff frei, der den Reaktor gasförmig verläßt. Auch gegen Reaktionsende werden die Temperaturen für eine Alkalischmelze unterschritten, um die bekannte Korrosivität zu vermeiden. Die Reaktoren können dadurch in preiswerten Werkstoffen ausgeführt werden. Die Temperaturen nach Beendigung der Reaktionsphase liegen zwischen 250 bis 350 °C. Die Stockpunkte der reinen Seifen werden damit teilweise nicht überschritten. Ein Festwerden der Seife vor dem Reaktionsende wird durch den noch vorhandenen Alkohol vermieden. Mit Erreichen einer Ausbeute von 98 % und mehr, bezogen auf den eingesetzten Alkohol, steigt schließlich die Viskosität stark an. Erst dann wird dem Reaktionsgemisch ein inertes Verdünnungsmittel zugegeben, um es hantierbar zu halten und den Stockpunkt der Reaktionsprodukte zu erniedrigen. Dabei reichen schon geringe gelöste Wassermengen, etwa 1 bis 5 Gew.-%, bei Temperaturen oberhalb von 200 °C aus.

Der zur Viskositätserniedrigung nach Reaktionsende bei 250 bis 350 °C eingeblasene Wasserdampf verläßt zum größten Teil den Reaktionsraum dampfförmig und führt erst nach einer Kondensation und erneuten Verdampfung zur Abkühlung und Erhöhung der Wasserlöslichkeit.

Bei dem erfindungsgemäßen Verfahren handelt es sich also im Gegensatz zum Stand der Technik um eine Fest-Flüssig-Reaktion, bei der die Temperaturen für eine Alkalischmelze unterschritten werden. Es wird insbesondere vorgeschlagen, daß in der Verfahrensstufe a, nämlich der Überführung der Guerbetalkohole in die Alkalisalze der entsprechenden Monocarbonsäuren, das Alkalihydroxid im flüssigem Alkohol suspendiert ist.

Bei der erfindungsgemäßen Durchführung der Fest-Flüssig-Reaktion unter Zugabe eines inerten Verdünnungsmittels zur Erniedrigung der Viskosität geht man vorzugsweise von in kaltem Zustand in den Reaktor vorgelegtem Alkalihydroxid und Guerbetalkohol aus, heizt das Reaktionsgemisch allmählich von etwa Raumtemperatur auf Reaktionstemperatur auf und kühlt nach Abschluß der Reaktion durch Zugabe des Verdünnungsmittels ab. Dabei führt man die Reaktion vorzugsweise bei Temperaturen von höchstens 350 °C durch. Die Temperaturen können dabei etwa zwischen 250 und 350 °C liegen.

Zum Einsatz als Kühl- und Verdünnungsmittel wird in einer Ausgestaltung der Erfindung ein bei Reaktionstemperatur verdampfbarer Stoff vorgeschlagen, insbesondere Wasser. Wasser ist nämlich nicht nur sehr gut geeignet, den Reaktionsansatz flüssig zu halten, sondern es ermöglicht auch eine sehr schnelle Kühlung des Reaktionsgemisches auf Temperaturen unter 200 °C. In einer weiteren Ausführungsform der Erfindung wird daher vorgeschlagen, daß man das Reaktionsgemisch auf Temperaturen unter 200 °C abkühlt. Die Zugabe des Wassers kann dabei auf unterschiedliche Weise erfolgen. Besonders bewährt hat sich das Einblasen von Wasserdampf sowie das Einspritzen von flüssigem Wasser.

Die Zugabe von 5 bis 30 Gew.-% Wasser zum Reaktionsgemisch reicht aus, um die gebildeten Seifen auch bei Raumtemperatur flüssig zu halten. Nach der Spaltung der Seifen kann das eingebrachte Wasser durch eine einfache Phasentrennung vollständig entfernt werden.

Bei der Abkühlung des Reaktionsgemisches durch Zugabe von Wasser verdampft der größere Teil des Wassers entsprechend dem Phasengleichgewicht zwischen Seife und Wasser. Das verdampfte Wasser wird in einer anderen vorteilhaften Ausführungsform kondensiert und in das Reaktionsgemisch zurückgeführt.

In einer anderen Ausführung des erfindungsgemäßen Verfahrens wird die Reaktion unter Überdruck durchgeführt, insbesondere unter einem Druck bis zu 10 bar, um die Gasgeschwindigkeit des entstehenden Wasserstoffs und damit das Aufschäumen zu verringern. Die Reaktion wird vorzugsweise unter Inertgas, z. B. Stickstoffgas, durchgeführt.

Die Wirtschaftlichkeit des Verfahrens wird außerdem erhöht, wenn man den in der ersten Verfahrensstufe, der Überführung der Guerbetalkohole in die entsprechenden Monocarbonsäuren, enstehenden Reaktionswasserstoff auffängt und verwertet. Er kann dabei insbesondere als Brenngas verwendet werden.

Nach der Umsetzung des Reaktionsgemisches zu den Salzen der verzweigten Monocarbonsäuren werden die entstandenen Seifen gespalten. Da im erfindungsgemäßen Verfahren lediglich stöchiometrische Mengen an Alkali eingesetzt werden, braucht die Seife im Reaktor nicht vom Alkali getrennt zu werden, sondern kann zur Seifenspaltung im Reaktor verbleiben.

Die Seifenspaltung kann wie in der Literatur beschrieben durchgeführt werden. Dazu kann unter Normaldruck z. B. Schwefelsäure oder Salzsäure eingesetzt werden. Aber auch eine Seifenspaltung unter Druck ist möglich und liegt im Rahmen des erfindungsgemäßen Verfahrens.

### Beispiele

Die nachfolgenden Beispiele sollen den Gegenstand der Erfindung näher erläutern, ohne ihn darauf zu beschränken.

### Beispiel 1: Herstellung von Isopalmitinsäure

Zur Herstellung wurde ein Rührreaktor verwendet. Die Peripherie besteht aus einem Dephlegmator, einem Kondensator und einem Phasenabscheider.

In den Reaktor wurden im kalten Zustand
- 8 000 kg: 2-Hexyldecanol
- 1 280 kg: NaOH
vorgelegt und unter Normaldruck aufgeheizt. Ab einer Reaktortemperatur von 230 °C begann die Reaktion unter H₂-Bildung. Bei Erreichen von 310 °C ließ die H₂-Bildung nach und die Viskosität stieg stark an. Durch Einblasen von Dampf mit einer Temperatur von 140 °C über ein Tauchrohr begann nun die Kühlphase. Ein Teil des Dampfes verblieb im Reaktor und sorgte für die Viskositätserniedrigung. Entsprechend dem Phasengleichgewicht Seife-Wasser verließ der größere Teil des Wassers den Reaktor dampfförmig und wurde im Dephlegmator kondensiert. Das Kondensat lief in den Reaktor zurück und entzog dem Reaktor über Verdampfung wiederum Wärme. Die entstandene Seifenlösung wurde nun mit 1 500 kg H₂SO₄ bei 80 °C zur Isopalmitinsäure gespalten.

Es entstanden ca. 7 800 kg Reaktionsprodukt mit einer Säurezahl SZ von 200 und einer Hydroxylzahl OHZ < 3. Während der Reaktionsphase wurden ca. 400 kg Destillat aufgefangen, das in weiteren Ansätzen als Einsatzstoff verwendet werden kann.

### Beispiel 2: Herstellung von Isopalmitinsäure unter erhöhtem Druck

Die Einsatzstoffe wurden wie in Beispiel 1 beschrieben vorgelegt, jedoch wurde der Reaktor vor dem Aufheizen mit einem Druck von 4 bar Stickstoff beaufschlagt. Das weitere Verfahren wurde wie in Beispiel 1 durchgeführt und 8 150 kg Produkt (SZ 201, OHZ < 3) und ca. 50 kg Destillat wurden erhalten.

### Beispiel 3: Herstellung von Isopalmitinsäure mit vorheriger Guerbetisierung

In einem 350 l Reaktor wurde 180 kg Octanol, 4,5 kg KOH und 80 g ZnO vorgelegt. Der Reaktor wurde auf 200 °C aufgeheizt. Nach Reaktionsbeginn wurde nach dem Kondensator ein Octanol-Wasser-Gemisch abgetrennt und die Alkoholphase in den Reaktor zurückgeführt. Die Reaktortemperatur stieg im Verlauf der Reaktion auf bis zu 250 °C an. In dem Reaktionsgemisch befand sich nach der Beendigung der Wasserausschleusung laut GC-Analyse ca. 8 % Octanol, 78 % 2-Hexyldecanol und ca. 8 % 2-4 Dihexyldodecanol. Durch Destillation bei 240 °C und 100 mbar wurde der Octanolgehalt auf unter 1 % gesenkt. Nach Entspannen auf Normaldruck wurden 24 kg NaOH in den Reaktor dosiert und der Ansatz wie in Beispiel 1 weitergeführt.

Es ergaben sich aus diesem Versuch 135 kg Produkt mit einer SZ von 205 und einer OHZ < 3. In der Destillation wurden 25,2 kg abgetrennt, die zu gleichen Teilen aus Octanol und Guerbetalkohol bestanden. Das Destillat kann als Einsatzstoff für weitere Ansätze verwendet werden.

### Beispiel 4: Herstellung von Isotridecylansäure

In den 350 l Reaktor wurden 180 kg Isotridecylalkohol und 33,6 kg NaOH eingesetzt und unter einem N₂-Druck von 6 bar auf 340 °C aufgeheizt. Ansonsten wurde das Verfahren wie unter Beispiel 1 durchgeführt. Ein Produkt mit einer SZ von 240 und einer OHZ von 6,3 wurde erhalten.

### Beispiel 5: Herstellung von Isocerotinsäure

In den Reaktor wurden 180 kg Lauryl- und Myristylalkohol zu gleichen Teilen zusammen mit 1,34 kg KOH und 80 g ZnO vorgelegt. Mit einem Verfahren entsprechend Beispiel 3 ergaben sich in der Destillation ca. 25 kg Gemischalkohol, der aus Monomeren und Dimeren zu gleichen Teilen bestand. Das nach der Spaltung erhaltene Produkt (145 kg) hatte eine SZ = 148 und eine OHZ < 2. Nach GC-Anlayse bestand dieses Gemisch zu 19 % aus Isotetracosansäure, zu 51 % aus Iso-Hexacosansäure (Isocerotinsäure) und zu 25 % aus Iso-Octacosansäure.

### Beispiel 6: Herstellung von Isopalmitinsäure (Seifenspaltung mit Kohlendioxid)

Das Verfahren wurde, wie in Beispiel 1 beschrieben, bis zur Seifenlösung durchgeführt. Anschließend wurde das Produkt im Verhältnis von 2 Teilen Wasser zu 1 Teil Seife verdünnt und in einen Autoklaven eingesetzt. Der Ansatz wurde bei 30 °C unter 60 bar CO₂ 2 Stunden gerührt. Nach einer Phasentrennung wurde in der Fettphase eine SZ von 189,2 gemessen.

## Patentansprüche

1. Verfahren zum Herstellen α-verzweigter aliphatischer Monocarbonsäuren mit 12 bis 48 Kohlenstoffatomen, wobei man
a) α-verzweigte aliphatische einwertige Alkohole (Guerbetalkohole) in Gegenwart von Alkalihydroxid in die Alkalisalze der entsprechenden α-verzweigten aliphatischen Monocarbonsäuren überführt und
b) aus den Alkalisalzen durch Seifenspaltung die α-verzweigten aliphatischen Monocarbonsäuren freisetzt,
**dadurch gekennzeichnet**,
daß man die Verfahrensstufe a als Reaktion zwischen festem Alkalihydroxid und flüssigem Alkohol durchführt und dem Reaktionsgemisch nach Abschluß dieser Reaktion ein inertes Verdünnungsmittel zum Absenken der Viskosität zugibt, wobei man das Verfahren insbesondere kontinuierlich durchführt.

2. Verfahren nach Anspruch 1,
**dadurch gekennzeichnet**,
daß in der Verfahrensstufe a das Alkalihydroxid im flüssigen Alkohol suspendiert ist.

3. Verfahren nach Anspruch 1 oder 2,
**dadurch gekennzeichnet**,
daß man in der Verfahrensstufe a das Reaktionsgemisch allmählich von etwa Raumtemperatur auf Reaktionstemperatur aufheizt und nach Abschluß der Reaktion durch Zugabe des Verdünnungsmittels abkühlt.

4. Verfahren nach Anspruch 3,
**dadurch gekennzeichnet**,
daß man die Reaktion bei Temperaturen von höchstens 350 °C durchführt.

5. Verfahren nach einem der Ansprüche 1 bis 4,
**dadurch gekennzeichnet**,
daß man als Kühl- und Verdünnungsmittel einen bei Reaktionstemperatur verdampfbaren Stoff einsetzt.

6. Verfahren nach Anspruch 5,
**dadurch gekennzeichnet**,
daß man als Kühl- und Verdünnungsmittel Wasser einsetzt.

7. Verfahren nach Anspruch 6,
**dadurch gekennzeichnet**,
daß man das Reaktionsgemisch auf Temperaturen unter 200 °C abkühlt.

8. Verfahren nach Anspruch 6 oder 7,
**dadurch gekennzeichnet**,
daß man Wasserdampf einbläst.

9. Verfahren nach Anspruch 6 oder 7,
**dadurch gekennzeichnet**,
daß man flüssiges Wasser einspritzt.

10. Verfahren nach einem der Ansprüche 6 bis 9,
**dadurch gekennzeichnet**,
daß man 5 bis 30 Gew.-% Wasser dem Reaktionsgemisch zugibt.

11. Verfahren nach einem der Ansprüche 6 bis 10,
**dadurch gekennzeichnet**,
daß man das verdampfte Wasser kondensiert und in das Reaktionsgemisch zurückführt.

12. Verfahren nach einem der Ansprüche 1 bis 11,
**dadurch gekennzeichnet**,
daß man die Verfahrensstufe a unter Überdruck durchführt.

13. Verfahren nach einem der Ansprüche 1 bis 12,
**dadurch gekennzeichnet**,
daß man den in der Verfahrensstufe a entstehenden Wasserstoff auffängt und verwertet und insbesondere als Brenngas verwendet.

## Claims

1. A process for the production of α-branched aliphatic monocarboxylic acids containing 12 to 48 carbon atoms in which
a) α-branched aliphatic monohydric alcohols (Guerbet alcohols) are converted into the alkali metal salts of the corresponding α-branched aliphatic monocarboxylic acids in the presence of alkali metal hydroxide and
b) the α-branched aliphatic monocarboxylic acids are released from the alkali metal salts by soap decomposition,
characterized in that stage a of the process is carried out as a reaction between solid alkali metal hydroxide and liquid alcohol and an inert diluent is added to the reaction mixture on completion of this reaction to reduce the viscosity, the process being carried out in particular continuously.

2. A process as claimed in claim 1, characterized in that the alkali metal hydroxide is suspended in the liquid alcohol in stage a.

3. A process as claimed in claim 1 or 2, characterized in that the reaction mixture is gradually heated from around room temperature to the reaction temperature in stage a and, on completion of the reaction, is cooled by addition of the diluent.

4. A process as claimed in claim 3, characterized in that the reaction is carried out at temperatures of at most 350°C.

5. A process as claimed in any of claims 1 to 4, characterized in that a substance evaporating at the reaction temperature is used as the coolant and diluent.

6. A process as claimed in claim 5, characterized in that water is used as the coolant and diluent.

7. A process as claimed in claim 6, characterized in that the reaction mixture is cooled to temperatures below 200°C.

8. A process as claimed in claim 6 or 7, characterized in that steam is introduced.

9. A process as claimed in claim 6 or 7, characterized in that liquid water is sprayed in.

10. A process as claimed in any of claims 6 to 9, characterized in that 5 to 30% by weight of water is added to the reaction mixture.

11. A process as claimed in any of claims 6 to 10, characterized in that the evaporated water is condensed and returned to the reaction mixture.

12. A process as claimed in any of claims 1 to 11, characterized in that stage a is carried out under excess pressure.

13. A process as claimed in any of claims 1 to 12, characterized in that the hydrogen formed in stage a is collected and used, more particularly as a combustible gas.

## Revendications

1. Procédé pour la préparation d'acides monocarboxyliques aliphatiques ramifiés en position α, contenant de 12 à 48 atomes de carbone, dans lequel
a) on transforme des alcools monovalents aliphatiques ramifiés en position α (alcools de Guerbet), en présence d'hydroxyde alcalin, en sels alcalins des acides monocarboxyliques aliphatiques correspondants ramifiés en position α, et
b) on libère des sels alcalins les acides monocarboxyliques aliphatiques ramifiés en position α par scission de savon,
caractérisé
en ce qu'on effectue l'étape opératoire a) sous forme d'une réaction entre l'hydroxyde alcalin solide et l'alcool liquide, et on ajoute au mélange réactionnel, une fois que cette réaction est arrivée à son terme, un diluant inerte pour diminuer la viscosité, en effectuant le procédé en particulier en continu.

2. Procédé selon la revendication 1,
caractérisé
en ce que, dans l'étape opératoire a), on met l'hydroxyde alcalin en suspension dans l'alcool liquide.

3. Procédé selon la revendication 1 ou 2,
caractérisé
en ce que, dans l'étape opératoire a), on chauffe progressivement le mélange réactionnel depuis approximativement la température ambiante jusqu'à la température réactionnelle et on le refroidit, une fois que la réaction est arrivée à son terme, par addition du diluant.

4. Procédé selon la revendication 3,
caractérisé
en ce qu'on effectue la réaction à des températures maximales de 350°C.

5. Procédé selon l'une quelconque des revendications 1 à 4,
caractérisé
en ce qu'on met en oeuvre, à titre de réfrigérant ou de diluant, une substance apte à s'évaporer à la température réactionnelle.

6. Procédé selon la revendication 5,
caractérisé
en ce que, à titre de réfrigérant et de diluant, on met en oeuvre de l'eau.

7. Procédé selon la revendication 6,
caractérisé
en ce qu'on refroidit le mélange réactionnel à des températures inférieures à 200°C.

8. Procédé selon la revendication 6 ou 7,
caractérisé
en ce qu'on insuffle de la vapeur d'eau.

9. Procédé selon la revendication 6 ou 7,
caractérisé
en ce qu'on injecte de l'eau liquide.

10. Procédé selon l'une quelconque des revendications 6 à 9,
caractérisé
en ce qu'on ajoute de l'eau au mélange réactionnel à concurrence de 5 à 30% en poids.

11. Procédé selon l'une quelconque des revendications 6 à 10,
caractérisé
en ce qu'on condense l'eau évaporée et on la recycle dans le mélange réactionnel.

12. Procédé selon l'une quelconque des revendications 1 à 11,
caractérisé
en ce qu'on effectue l'étape opératoire a) sous une pression supérieure.

13. Procédé selon l'une quelconque des revendications 1 à 12,
caractérisé
en ce qu'on récolte et on utilise l'hydrogène obtenu à l'étape opératoire a) et on l'utilise en particulier comme combustible gazeux.
